# EUROPEAN PATENT APPLICATION

(11) **EP 2 952 131 A1**
(43) Date of publication of application: **09.12.2015**
(21) Application number: 14746162.8
(22) Date of filing: 28.01.2014
(51) Int. Cl.: A61B 1/00, A61B 1/06, G02B 23/26

(54) **SCANNING OBSERVATION DEVICE AND CONTROL METHOD THEREFOR**

(30) Priority: 29.01.2013 JP 2013014270
(71) Applicant: Olympus Corporation, Shibuya-ku Tokyo 151-0072 (JP)
(72) Inventor: NISHIMURA, Junichi, Tokyo 151-0072 (JP)
(74) Representative: Gunzelmann, Rainer
(86) International application number: PCT/JP2014/051823
(87) International publication number: WO 2014/119563

(57) **Abstract**

To stabilize vibration of an optical fiber by an actuator regardless of a drive frequency thereof in fiber-scanning observation. A scanning observation apparatus (1) includes: an optical fiber (7) that emits illumination light; a scanning section (3) that two-dimensionally scans an observation object with the illumination light by giving vibration to a distal end of the optical fiber (7); a waveform generation section (9) that generates a vibration waveform of the distal end of the optical fiber (7) by the scanning section (3); a sampling section (10) that samples a vibration waveform generated by the waveform generation section (9) and instructs the scanning section (3) on the vibration waveform; and a control section (6) that supplies a drive frequency and a frame period to the waveform generation section (9), and a scanning sampling rate to the sampling section (10), wherein the control section (6) calculates the frame period by dividing the number of vibrations in one frame period input thereto by the drive frequency, and calculates the scanning sampling rate so as to be an integer multiple of the drive frequency.

## Description

### {Technical Field}

The present invention relates to a scanning endoscope and a control method thereof.

### {Background Art}

Conventionally, a scanning endoscope has been known which two-dimensionally scans an observation object with illumination light emitted from a distal end of an optical fiber by vibrating the distal end of the optical fiber in orientations by an actuator such as a piezoelectric device and changing an amplitude of the vibration, and detects light (e.g., reflected light or fluorescence) returning from each irradiated position to form an image (e.g., see Patent Literature 1).

In the scanning endoscope, a continuous scanning waveform is formed by D/A-converting a series of amplitude coordinate values calculated in a controller, and at the same time, light returning in a direction of the scanning endoscope from each position of the observation object and detected by an optical sensor is A/D-converted to construct an image.

### {Citation List}

### {Patent Literature}

{PTL 1}
Japanese Unexamined Patent Application, Publication No. 2009-240601

### {Summary of Invention}

### {Technical Problem}

However, since a frame rate, a drive frequency of the actuator, and a sampling rate are optionally set in the conventional scanning endoscope, it is difficult to synchronize driving of the actuator and signal acquisition. Thus, the vibration of the optical fiber by the actuator becomes unstable.

For example, in a case in which waveform data is generated by employing two frames as one set when a scanning trajectory of illumination light is formed in a spiral shape, a waveform becomes discontinuous at a frame break point if a base waveform is not precisely fitted within the two frames. Thus, the vibration of the optical fiber by the actuator becomes unstable in some cases.

The present invention has been made in view of the above circumstances, and an object thereof is to provide a scanning observation apparatus capable of stabilizing vibration of an optical fiber by an actuator regardless of a drive frequency of the actuator, and a control method thereof.

### {Solution to Problem}

In order to achieve the above object, the present invention provides the following solutions.

One aspect of the present invention provides a scanning observation apparatus including: an optical fiber that emits illumination light; a scanning section that two-dimensionally scans an observation object with the illumination light by giving vibration to a distal end of the optical fiber; a waveform generation section that generates a vibration waveform of the distal end of the optical fiber by the scanning section; a sampling section that samples the vibration waveform generated by the waveform generation section and instructs the vibration waveform to the scanning section; and a control section that supplies a drive frequency and a frame period to the waveform generation section and supplies a scanning sampling rate to the sampling section, wherein the control section calculates the frame period by dividing the number of vibrations in one frame period input thereto by the drive frequency, and calculates the scanning sampling rate so as to be an integer multiple of the drive frequency.

In accordance with the present aspect, when the drive frequency and the number of vibrations in one frame period are input, the control section calculates the frame period, and supplies the calculated frame period and the input drive frequency to the waveform generation section, and the control section calculates the scanning sampling rate that is an integer multiple of the drive frequency and supplies the scanning sampling rate to the sampling section. In the waveform generation section, the vibration waveform is generated based on the drive frequency and the frame period supplied from the control section.

In the sampling section, the vibration waveform generated in the waveform generation section is sampled at the sampling rate supplied from the control section, and instructed to the scanning section. The scanning section receiving the instruction vibrates the distal end of the optical fiber such that the distal end of the optical fiber is arranged at an instructed position. Accordingly, the distal end of the optical fiber is vibrated so as to achieve the vibration waveform generated in the waveform generation section, and the observation object is two-dimensionally scanned with the illumination light emitted from the distal end of the optical fiber along a trajectory according to the vibration waveform.

In this case, since the control section calculates the frame period by multiplying the drive frequency and the number of vibrations in one frame period together, it is possible to generate the vibration waveform such that an entire waveform of vibrations of the optical fiber is completely fitted within the frame period. Also, since the scanning sampling rate is calculated so as to be an integer multiple of the drive frequency, it is possible to prevent the vibration of the distal end of the optical fiber achieved by the scanning section from becoming discontinuous at a break point between frame periods. As a result, the vibration of the optical fiber by the actuator can be stabilized regardless of the drive frequency of the actuator.

In the above aspect, the scanning observation apparatus may further include: an optical detection section that detects light emanated from a scanning position on the observation object in response to the illumination light emitted from the optical fiber; and an image generation section that samples an intensity signal of the light detected by the optical detection section at an image sampling rate and correlates the intensity signal with the scanning position, wherein the control section may calculate the image sampling rate so as to be an integer multiple of the drive frequency, and supply the image sampling rate to the image generation section.

Accordingly, since the light emitted from the scanning position is detected by the optical detection section, and the image generation section samples the intensity signal of the light at the image sampling rate and correlates the intensity signal with the scanning position, an image of the observation object is generated. In this case, by setting the image sampling rate to an integer multiple of the drive frequency, it is possible to fit all sampling intervals within the frame period, and it is possible to prevent distortion of the generated image by preventing a sampling interval shorter than the other sampling intervals from being produced at a break point between frame periods.

Also, in the above aspect, the control section may calculate the image sampling rate by multiplying the scanning sampling rate by an integer.

Accordingly, it is possible to acquire intensity information of the light for generating an image at a sampling interval sufficiently smaller than the scanning sampling rate, and it is possible to improve resolution.

Also, in the above aspect, the control section may calculate the scanning sampling rate by rounding up or down a value obtained by dividing an optional tentative sampling rate by the drive frequency to convert the value into an integer, and multiplying the value converted into an integer by the drive frequency.

Accordingly, a sampling rate close to a desired value can be employed by inputting the desired value as the optional tentative sampling rate. Thus, unstable vibration of the optical fiber can be prevented and the distortion of the generated image can be reduced.

Also, another aspect of the present invention provides a method for controlling a scanning observation apparatus, the method being applied to a scanning observation apparatus including: an optical fiber that emits illumination light; a drive section that two-dimensionally scans an observation object with the illumination light by giving vibration to a distal end of the optical fiber; a waveform generation section that generates a vibration waveform of the distal end of the optical fiber by the drive section; a sampling section that samples the vibration waveform generated by the waveform generation section and instructs the drive section on the vibration waveform; and a control section that supplies a drive frequency and a frame period to the waveform generation section and supplies a scanning sampling rate to the sampling section, wherein the control section calculates the frame period by dividing the number of vibrations in one frame period input thereto by the drive frequency, calculates the scanning sampling rate so as to be an integer multiple of the drive frequency, and outputs the calculated scanning sampling rate as a sampling instruction signal.

In accordance with the present aspect, when the drive frequency and the number of vibrations in one frame period are input, the control section calculates the frame period, and supplies the calculated frame period and the input drive frequency to the waveform generation section, and the control section calculates the scanning sampling rate that is an integer multiple of the drive frequency and transmits the sampling instruction signal to the sampling section. Therefore, in accordance with the control method of the present aspect, in a scanning section of the scanning observation apparatus receiving an instruction from the control section, a vibration waveform according to the instruction signal is generated in the waveform generation section, the vibration is given to the optical fiber so as to achieve the vibration waveform, and the observation object is two-dimensionally scanned with the illumination light emitted from the distal end of the optical fiber along a trajectory according to the vibration waveform.

### {Advantageous Effects of Invention}

The present invention provides an effect that the vibration of the optical fiber by the actuator can be stabilized regardless of the drive frequency of the actuator in the scanning observation apparatus and the control method.

### {Brief Description of Drawings}

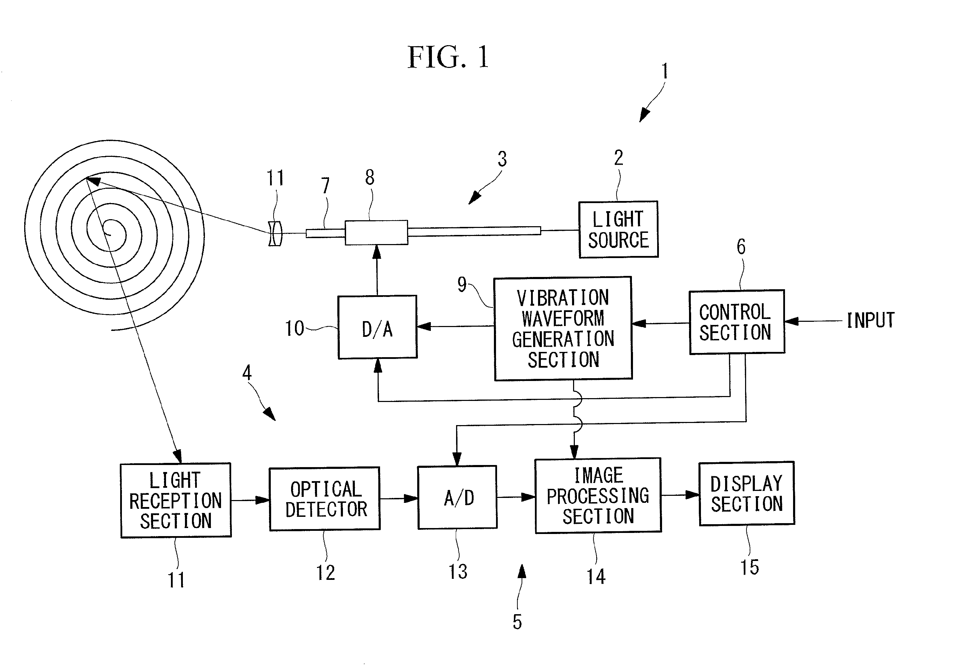

{Fig. 1}
   Fig. 1 is a block diagram illustrating a scanning endoscope according to one embodiment of the present invention.
{Fig. 2}
   Fig. 2(a) is a view illustrating one example of a scanning trajectory of illumination light, and Fig. 2(b) is a view illustrating one example of a vibration waveform of an optical fiber for achieving the scanning trajectory in the scanning endoscope in Fig. 1.
{Fig. 3}
   Fig. 3 is a circuit diagram illustrating a control section of the scanning endoscope in Fig. 1.

### {Description of Embodiments}

A scanning endoscope (a scanning observation apparatus) 1 according to one embodiment of the present invention will be described below with reference to the drawings.

As shown in Fig. 1, the scanning endoscope 1 according to the present embodiment includes a light source 2 that produces illumination light, a scanning section 3 that two-dimensionally scans a surface of an observation object with the illumination light from the light source 2, an optical detection section 4 that detects light produced from a scanning position with the illumination light by the scanning section 3, an image generation section 5 that generates an image of the observation object based on the light detected by the optical detection section 4, and a control section 6 that controls the sections.

The scanning section 3 includes an optical fiber 7 that guides the light from the light source 2 and emits the light from a distal end, an actuator (a drive section) 8 that vibrates the distal end of the optical fiber 7, a vibration waveform generation section 9 that generates a vibration waveform of the distal end of the optical fiber 7 by the actuator 8, and a D/A converter (a sampling section) 10 that samples the vibration waveform generated by the vibration waveform generation section 9 at a predetermined sampling rate (a scanning sampling rate) and outputs the vibration waveform to the actuator 8.

The actuator 8 is composed of, for example, two piezoelectric devices (not shown), which are attached to an outer peripheral surface in the vicinity of the distal end of the optical fiber 7 at positions circumferentially different by 90° from each other. Accordingly, the actuator 8 can curve (swing) the optical fiber 7 independently in two directions (an X direction and a Y direction) perpendicular to a longitudinal direction and perpendicular to each other, and thereby two-dimensionally moves the distal end of the optical fiber 7 to perform two-dimensional scanning with the illumination light.

The vibration waveform generation section 9 generates respective vibration waveforms for driving the two piezoelectric devices constituting the actuator 8. The vibration waveform generation section 9 stores amplitudes of vibrations in the X direction and the Y direction, and a phase difference between the vibrations in the X direction and the Y direction, and when a frame period and a drive frequency are input, the vibration waveform generation section 9 generates the respective vibration waveforms for the two piezoelectric devices.

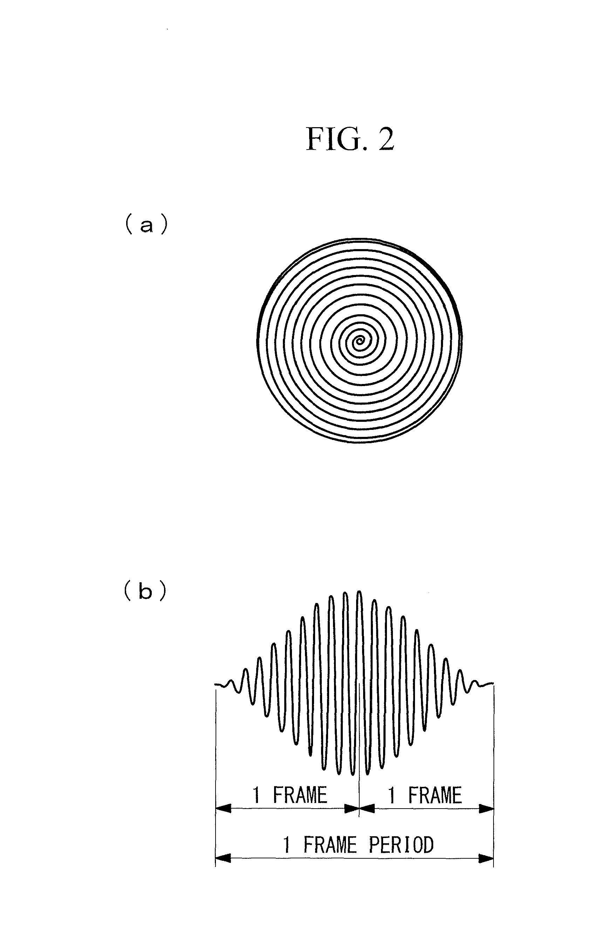

In the present embodiment, the vibration waveform generation section 9 generates a vibration waveform that causes the distal end of the optical fiber 7 to draw, for example, a swirling (or spiral) trajectory as shown in Fig. 2(a). Fig. 2(b) shows each of vibration waveforms in the X direction and the Y direction generated by the respective piezoelectric devices at this time, and the vibration waveforms have a phase difference of about 90°.

By vibrating from a center portion to an outermost edge portion of the swirling trajectory, the swirling trajectory that spreads so as to fill a predetermined region on the observation object as shown in Fig. 2(a) is obtained, so that a single image can be constructed. Therefore, a period from a minimum amplitude to a maximum amplitude in Fig. 2(a), or a period from the maximum amplitude to the minimum amplitude is one frame for generating an image. However, since two frames are required as a period in which the distal end of the optical fiber 7 returns to the minimum amplitude from the minimum amplitude, the two frames are expressed as a frame period in the present specification.

The optical detection section 4 includes a light reception section 11, such as a plurality of optical fibers, a distal end surface of which is arranged toward a front, and an optical detector 12, such as a photodiode or a photomultiplier tube, that converts light received by the light reception section 11 to an electric signal, for example, in the vicinity of the distal end of the optical fiber 7 that emits the illumination light.

The image generation section 5 includes an A/D converter 13 that samples the electric signal converted by the optical detector 12 at a predetermined sampling rate (an image sampling rate), and an image processing section 14 that generates an image by correlating an intensity signal of the light output from the A/D converter 13 with a scanning position with the illumination light at a point of time of the light reception. In the drawings, reference numeral 15 denotes a display section that displays the generated image.

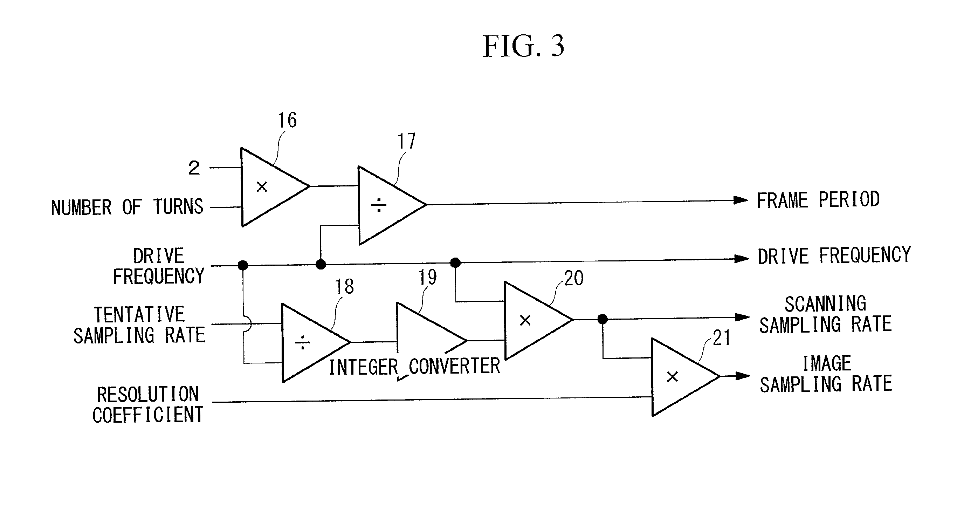

As shown in Fig. 3, the number of turns, a drive frequency, a tentative sampling rate, and a resolution coefficient are input to the control section 6, and the control section 6 outputs the frame period, the drive frequency, the scanning sampling rate, and the image sampling rate.

The number of turns is the number of vibrations of the optical fiber 7 in one frame. In the control section 6, the number of vibrations in the frame period composed of two frames is calculated by multiplying the number of turns by two in a multiplier 16.

The control section 6 calculates the frame period by dividing the doubled number of turns input thereto by the drive frequency similarly input thereto in a divider 17. The frame period calculated as described above and the input drive frequency are transmitted to the vibration waveform generation section 9.

The control section 6 also calculates the scanning sampling rate by dividing the tentative sampling rate input thereto by the drive frequency similarly input thereto in a divider 18, rounding down a value obtained as a result of the division to convert the value into an integer in an integer conversion section 19, and thereafter multiplying the integer and the input drive frequency together in a multiplier 20.

The control section 6 further calculates the image sampling rate by multiplying the calculated scanning sampling rate and the input resolution coefficient together in a multiplier 21.

The resolution coefficient is an optional integer, and as the resolution coefficient has a larger numerical value, an image of higher resolution can be acquired.

An operation of the scanning endoscope 1 according to the present embodiment having the above configuration will be described below.

To acquire an image of the observation object by using the present embodiment, the number of turns, the drive frequency, the tentative sampling rate, and the resolution coefficient are input to the control section 6, and the distal end of the optical fiber 7 of the scanning section 3 is arranged toward the observation object. In this state, the light source 2 produces light, and the control section 6 is operated to control the vibration waveform generation section 9.

That is, the control section 6 calculates the frame period from the number of turns and the drive frequency input thereto, and inputs the calculated frame period to the vibration waveform generation section 9 together with the drive frequency. The vibration waveform generation section 9 generates and outputs the vibration waveform for driving the actuator 8 in the X direction and the Y direction based on the frame period and the drive frequency input thereto, and the amplitudes and the phase difference in the X direction and the Y direction that are previously stored.

In this case, since the frame period is generated by dividing the doubled number of turns by the drive frequency, it is possible to generate the vibration waveform such that an entire waveform of vibrations of the optical fiber 7 vibrated at the drive frequency of the actuator 8 and equal to the doubled number of turns is closely fitted within the frame period.

Also, since the control section 6 calculates the scanning sampling rate by rounding down the value obtained by dividing the tentative sampling rate by the drive frequency to convert the value into an integer, and thereafter multiplying the integer and the drive frequency together again, the scanning sampling rate can be set to an integer multiple of the drive frequency. The calculated scanning sampling rate is input as a sampling instruction signal to the D/A converter 10, so that the vibration waveform generated in the vibration waveform generation section 9 is evenly sampled in the frame period, and is output to the actuator 8.

Therefore, it is possible to prevent the vibration of the distal end of the optical fiber 7 achieved by the actuator 8 from becoming discontinuous at a break point between frame periods. As a result, there is an advantage that the vibration of the optical fiber 7 by the actuator 8 can be stabilized regardless of the drive frequency of the actuator 8.

Also, the light emitted from the irradiated position with the illumination light emitted from the optical fiber 7 is received by the light reception section 11, and is detected by the optical detector 12. When the image sampling rate output from the control section 6 is input to the A/D converter 13, the output signal from the optical detector 12 is sampled. Since the image sampling rate is set to an integer multiple of the scanning sampling rate, the image sampling rate is an integer multiple of the drive frequency similarly to the scanning sampling rate.

Therefore, intensity information of the light from the observation object is evenly sampled in the frame period, and is stored in correlation with scanning position information in the sampling input from the vibration waveform generation section 9. The image of the observation object can be thereby generated. The generated image is displayed on the display section 15.

In this case, by setting the image sampling rate to an integer multiple of the drive frequency, there is an advantage that it is possible to fit all sampling intervals within the frame period, and it is possible to prevent distortion of the generated image by preventing a sampling interval shorter than the other sampling intervals from being produced at a break point between frame periods.

Although the case in which the distal end of the optical fiber 7 is vibrated so as to draw the swirling trajectory as shown in Fig. 2(a) is described in the present embodiment, the present invention may be also applied to a case in which any other scanning patterns in which the fiber is periodically vibrated, such as a raster scan method and a Lissajous scan method, are used instead of the above case. Also, as the observation apparatus, the present invention may be applied to various observation apparatuses of small and/or small-diameter light illumination type other than the endoscope. Also, the present invention is not limited to piezoelectric fiber driving, and may be applied to any other driving methods (e.g., an electromagnetic driving method) in which vibration is given to a distal end of a fiber or the like, and the fiber distal end is moved in a desired scanning pattern.

### {Reference Signs List}

- 1: Scanning endoscope (scanning observation apparatus)
- 4: Optical detection section
- 5: Image generation section
- 6: Control section
- 7: Optical fiber
- 8: Actuator (driving section)
- 9: Vibration waveform generation section (waveform generation section)
- 10: D/A converter (sampling section)

## Claims

1. A scanning observation apparatus comprising:
an optical fiber that emits illumination light;
a drive section that two-dimensionally scans an observation object with the illumination light by giving vibration to a distal end of the optical fiber;
a waveform generation section that generates a vibration waveform of the distal end of the optical fiber by the drive section;
a sampling section that samples the vibration waveform generated by the waveform generation section and instructs the drive section on the vibration waveform; and
a control section that supplies a drive frequency and a frame period to the waveform generation section and supplies a scanning sampling rate to the sampling section,
wherein the control section calculates the frame period by dividing the number of vibrations in one frame period input thereto by the drive frequency, and calculates the scanning sampling rate so as to be an integer multiple of the drive frequency.

2. The scanning observation apparatus according to claim 1, further comprising:
an optical detection section that detects light emanated from a scanning position on the observation object in response to the illumination light emitted from the optical fiber; and
an image generation section that samples an intensity signal of the light detected by the optical detection section at an image sampling rate and correlates the intensity signal with the scanning position,
wherein the control section calculates the image sampling rate so as to be an integer multiple of the drive frequency, and supplies the image sampling rate to the image generation section.

3. The scanning observation apparatus according to claim 2, wherein the control section calculates the image sampling rate by multiplying the scanning sampling rate by an integer.

4. The scanning observation apparatus according to any of claims 1 to 3, wherein the control section calculates the scanning sampling rate by rounding up or down a value obtained by dividing an optional tentative sampling rate by the drive frequency to convert the value into an integer, and multiplying the value converted into an integer by the drive frequency.

5. A method for controlling a scanning observation apparatus, the method being applied to a scanning observation apparatus comprising:
an optical fiber that emits illumination light; a drive section that two-dimensionally scans an observation object with the illumination light by giving vibration to a distal end of the optical fiber; a waveform generation section that generates a vibration waveform of the distal end of the optical fiber by the drive section; a sampling section that samples the vibration waveform generated by the waveform generation section and instructs the drive section on the vibration waveform; and a control section that supplies a drive frequency and a frame period to the waveform generation section and supplies a scanning sampling rate to the sampling section,
wherein the control section calculates the frame period by dividing the number of vibrations in one frame period input thereto by the drive frequency, calculates the scanning sampling rate so as to be an integer multiple of the drive frequency, and outputs the calculated sampling rate as a sampling instruction signal.
